# EUROPÄISCHE PATENTANMELDUNG

(11) **EP 0 848 959 A2**
(43) Veröffentlichungstag der Anmeldung: **24.06.1998**
(21) Anmeldenummer: 97112345.0
(22) Anmeldetag: 18.07.1997
(51) Int. Cl.: A61L 15/44

(54) **Humantherapeutisches Erzeugnis zur Behandlung und/oder Vorbeugung von Krankheiten durch Dämpfe von ätherischen Ölen**

(30) Priorität: 20.11.1996 DE 29620182 U
(71) Anmelder: BB med. product GmbH, 47546 Kalkar (Niedermörmter) (DE)
(72) Erfinder: Beinio, Heinz Fred, 47551 Bedburg-Hau (DE)
(74) Vertreter: Herrmann-Trentepohl, Werner, Dipl.-Ing.

(57) **Zusammenfassung**

Bei einem humantherapeutischen Erzeugnis zur Behandlung und/oder Vorbeugung von Krankheiten durch Einatmen von Dämpfen ätherischer Öle eines Substrates, ist erfindungsgemäß ein Blatt (1) aus Zellstoff mit einer Mehrzahl von regelmäßigen Vertiefungen (5, 8), in denen das Substrat enthalten ist, und eine dampfdichte Hülle, in der das mit dem Substrat getränkte Zellstoffblatt (1) enthalten und aus der es zur Therapie entnehmbar ist, vorgesehen.

## Beschreibung

Die Erfindung betrifft ein humantherapeutisches Erzeugnis zur Behandlung und/oder Vorbeugung von Krankheiten durch Dämpfe von ätherischen Ölen gemaß dem Oberbegriff des Anspruches 1.

Das erfindungsgemäße humantherapeutische Erzeugnis bezieht sich insbesondere auf die Behandlung von und die Vorsorge gegen Erkältungskrankheiten, ist aber daneben auch geeignet, die Atemluft zu verbessern, um Beeinträchtigungen durch die Atemluft entgegenzuwirken. Dadurch laßt sich die Belindlichkeit empfindlicher Personen, u.a. von Asthmatikern, verbessern.

Für eine optimale Wirkung solcher Erzeugnisse kommt es einerseits darauf an, daß sie eine längere Behandlungsdauer ermöglichen. Andererseits soll die Behandlung möglichst ohne Beeinträchtigung der betroffenen und der gesunden Körperpartien erfolgen, um schädliche Nebenwirkungen gering zu halten.

Es ist bekannt, ätherische Öle in hoher Konzentration in Riechfläschchen als therapeutisches Erzeugnis zur Verfügung zu stellen. Da jedoch solche Substrate sich beeinträchtigend insbesondere auf Schleimhäute auswirken, die mit dem Substrat bei der Behandlung in direkte Berührung kommen, ist die Anwendung eines solchen Erzeugnisses problematisch. Substrate, die praktisch reine ätherische Öle darstellen, müssen daher vor ihrer Applikation in Dämpfe umgewandelt werden. Riechfläschchen sind hierfür wenig leistungsfähig, so daß in der Regel das Substrat meistens nach vorheriger Verdünnung angewandt wird. Insbesondere kommen dafür Inhaliergeräte in Betracht.

Es ist allerdings auch bekannt, Substrate ätherischer Öle unmittelbar äußerlich anzuwenden, indem das flüssige Substrat beispielsweise auf die Brust des Patienten gerieben wird. Die sich dann durch die Körperwärme bildenden Dämpfe werden von dem Patienten eingeatmet. Solche Verfahren sind wenig wirksam, da bei ihrer Anwendung Rücksicht auf die Empfindlichkeit der Haut genommen werden muß und außerdem die Überführung der Dämpfe in die Außenluft nur geringe Konzentrationen in der eingeatmeten Luft ermöglicht.

Die Erfindung geht einen anderen Weg, dessen Grundgedanke im Anspruch 1 wiedergegeben ist; weitere Merkmale der Erfindung sind Gegenstand der Unteransprüche.

Gemäß der Erfindung besteht das neue Erzeugnis aus einem Blatt oder Tuch aus Zellstoff, das in einer Mehrzahl von Vertiefungen der Oberfläche das Substrat enthält. Diese Vertiefungen sind deswegen einerseits zahlreich, andererseits aber von geringer Ausdehnung, so daß aufgrund physikalischer Kräfte, die sich in den Vertiefungen durch das flüssige Substrat abspielen, erhebliche Flüssigkeitsmengen auf einer vergleichsweise geringen Oberfläche des Blattes oder Tuches untergebracht werden können. Die Kräfte, welche das Substrat festhalten, sind zwar vergleichsweise gering. Das zeigt sich daran, daß man das Tuch auswringen kann, wodurch das flüssige Substrat frei wird und ausfließt. Andererseits kann aber deswegen das Substrat auch verhältnismäßig leicht verdampfen, was wegen der erheblichen Flüssigkeitsmenge in einer gegebenen Ausdehnung des Blattes zu einer vergleichsweise hohen Dampfkonzentration in der Umgebungsluft führt.

Die Applikation der Dämpfe mit dem erfindungsgemäßen Erzeugnis kann daher durch einfaches Auflegen des ausgebreiteten Blattes oder Tuches auf das Gesicht oder die Brust erfolgen oder das Tuch wird im Brustbereich beispielsweise an der Kleidung befestigt. Beeinträchtigungen der betroffenen Körperpartien durch unmittelbare Einwirkung des Substrates insbesondere auf Schleimhäute sind nicht zu befürchten. Andererseits ist wegen der hohen Konzentration der Dämpfe ein guter Behandlungs- bzw. Vorbeugungseffekt zu erreichen, da das Tuch über längere Zeit seine Wirksamkeit behält. Von besonderem Vorteil ist, daß das erfindungsgemäße Erzeugnis ein Substrat mit hoher Konzentration ätherischer Öle enthält, jedoch nicht eine zusätzliche Applikationsvorrichtung erfordert, sondern als solches zur Anwendung gebracht werden kann.

Vorzugsweise und zur Steigerung der Effektivität des neuen Erzeugnisses ist im Anspruch 2 vorgesehen, daß beiderseits des Blattes Vertiefungen angebracht sind, die zusammenwirken und dennoch geschlossen sind. Das bedeutet, daß die Vertiefungen einen dünnen Boden aufweisen und das Blatt zu einem geschlossenporigen Erzeugnis machen. Diese Vertiefungen bilden Näpfe, in denen sich erhebliche Flüssigkeitsmengen unterbringen lassen.

Für die Herstellung des erfindungsgemäßen Erzeugnisses steht im Vordergrund eine einfache Arbeitsweise. Dazu tragen die Merkmale des Anspruches 3 bei. Hierbei sind nämlich die Vertiefungen untereinander im wesentlichen identisch und in gleichen Abständen angeordnet. Sie lassen sich daher maschinell in ein Zellstoffblatt auf einfache Weise einbringen, beispielsweise einwalzen. Daher besteht eine zweckmäßige Ausführungsform der Erfindung, die im Anspruch 5 wiedergegeben ist, darin, die Vertiefungen in einem Prägevorgang in das Blatt einzubringen.

Andererseits ist die Zahl der Vertiefungen auch begrenzt, wenn optimale Flüssigkeitsmengen auf die beschriebene Weise in einem Zellstoffblatt festgehalten werden sollen. Es ist deshalb von Vorteil, wenn gemäß den Merkmalen des Anspruches 5 die Vertiefungen rechteckig begrenzt und mit ihren Seiten parallel zu den Blattkanten in Reihen angeordnet sind. Hierdurch werden in den beiden Flächendimensionen Abstände der Vertiefungen eingehalten, die ein Zusammenfließen der Flüssigkeiten in den Vertiefungen und ein Freiwerden größerer Flüssigkeitsmengen aus dem einmal mit der Flüssigkeit versehenen Zellstoffblatt verhindern.

Diese Wirkung läßt sich noch dadurch optimieren, daß gemäß Anspruch 6 die Vertiefungen "auf Lücke" angeordnet werden.

Als optimal haben sich in der Praxis Abmessungen der Vertiefungen herausgestellt, die für ätherische Öle in verhältnismäßig hoher Konzentration die Bemessungen aufweisen, die im Anspruch 7 wiedergegeben sind.

Das Material, aus dem das Zellstoffblatt besteht, bedarf seinerseits der Optimierung. Besonders geeignet ist sogenannter Fluffzellstoff, bei dem eine copolymere Kunststoffdispersion als Bindemittel verwendet wird. Ein solches Blatt wird insbesondere mit den kennzeichnenden Merkmalen des Anspruches 9 verwendet.

Wenn die Erfindung mit derartigem Material verwendet wird, ergibt sich ein verhältnismäßig dünnes Blatt, dessen Flächengewicht (Meßmethode 40.3-89) vergleichsweise gering ist, dessen Naßfestigkeit in Längs- und Querrichtung (Meßmethode 20.2-89) jedoch vergleichsweise hoch ist und dessen Absorption mit ca. 480 g/m2 (Meßmethode 10.1-72) erhebliche Substratmengen ermöglicht. Diese im Anspruch 10 wiedergegebenen Spezifikationen lassen sich weiter mit den Merkmalen des Anspruches 11 optimieren. Danach ist die Dehnung des Blattes (Meßmethode 20.2-89) in Längs- und in Querrichtung vergleichsweise gering, jedoch ist die Festigkeit im trockenen Zustand in Längs- und Querrichtung (Meßmethode 20.2-89) vergleichsweise hoch.

Mit derartigen Materialkonstanten läßt sich die Erfindung in Form eines Taschentuchs verwirklichen und hat dann die bevorzugten Abmessungen, die im Anspruch 12 wiedergegeben sind.

Die Unterbringung des Substrates auf dem Tuch und in den regelmäßigen Vertiefungen erfolgt im allgemeinen durch Tränkung. Da der Partialdruck der Dämpfe gegenüber Luft verhältnismäßig groß ist, muß das Blatt vor seiner Anwendung dampfdicht untergebracht werden. Mit den vorstehend beschriebenen Abmessungen der Materialkonstanten ist es möglich, das Blatt zu falten und in der dampfdichten Hülle raumsparend unterzubringen.

Als Substrat kommen ätherische Öle der im Anspruch 14 wiedergegebenen Art in Mischung, aber auch einzeln in Betracht.

Die Erfindung wird im folgenden anhand eines Ausführungsbeispieles näher erläutert; es zeigen
- Fig. 1: das erfindungsgemäße Erzeugnis unter Weglassung der dampfdichten Hülle schematisch und in Draufsicht,
- Fig. 2: einen Teilschnitt längs der Linie II-II der Fig. 1 und
- Fig. 3: eine Einzelheit der Fig. 1 an der mit dem Pfeil III gekennzeichneten Stelle.

Die Zeichnungen zeigen ein Blatt aus 100 %igem Fluffzellstoff, das mit 1 bezeichnet ist. Der Faserstoff macht ca. 80 Gew.% aus. Als Bindemittel dient eine copolymere Kunststoff-Dispersion in einem Verhältnis von 20 Gew.%. Das Material ist grundsätzlich brennbar. Der Glühverlust des Trockenrückstandes beträgt über 99 % (bestimmt gemäß DIN 38414 Teil 3). Er ist deponierbar und nach vorheriger Zerkleinerung auch kompostierbar. Die Reststoffgehalte des Materials sind sehr niedrig und liegen erheblich unterhalb der maximal zulässigen Grenzwerte nach der Klärschlammverordnung vom 25.06.1982 sowie unterhalb der Grenzwerte der Güterichtlinien für hochwertige Komposte der Bundesgütegemeinschaft Kompost e. V..

Das Erzeugnis ist daher absolut umweltfreundlich.

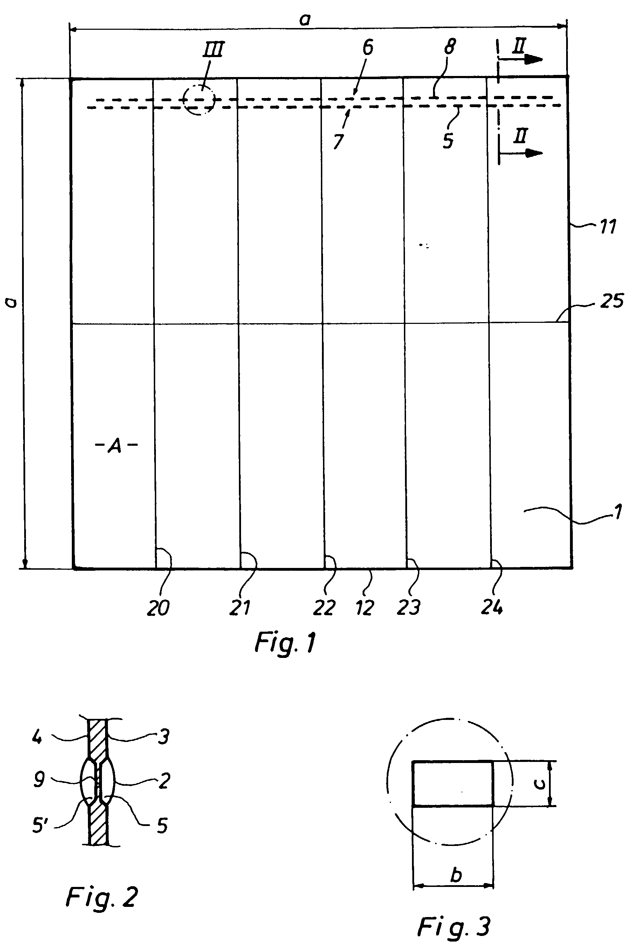

Das geringe Flächengewicht des Blattes ermöglicht eine Faltung - Falten 20 bis 24 in einer und Falte 25 in der dazu rechtwinkligen Richtung -, die es auf eine Teilfläche A von einem Zehntel der ausgebreiteten Fläche verkleinert. Ist das Tuch gefaltet, läßt es sich in einer dampfdichten Hülle unterbringen, die eine Bildung von Dämpfen und damit das vorzeitige Verdampfen ätherischer Öle 2 verhindert (Fig. 2).

Das Blatt ist im wesentlichen quadratisch, d.h. seine Seitenlängen a sind gleich und betragen in der Praxis beispielsweise 20 cm. Auf beiden Seiten 3,4 sind Vertiefungen 5 in mehreren Reihen, wie am Beispiel der Reihen 6, 7 zu zeigen, angebracht. Die Vertiefungen 5, 5' auf den Seiten 3 und 4 befinden sich unmittelbar über- und untereinander. Hierbei ist vorgesehen (Fig. 3), daß die Länge b der längeren Rechteckseiten der Vertiefungen 5, 8 gleich oder kleiner als 1 mm ist. Die Abstände sind größer und machen im Ausführungsbeispiel ca. 2 x b aus. Aufgrund der physikalischen Eigenschaften des Blattmaterials und der die ätherischen Öle enthaltenden Flüssigkeit ergeben sich in den Vertiefungen verhältnismäßig große Mengen des Substrates wie bei 2 in Fig. 2 schematisch angedeutet.

Die Vertiefungen 5 der Reihe 7 sind in der Lücke zwischen den Vertiefungen 8 der Reihe 6 angebracht, stehen also "auf Lücke". Auf diese Weise lassen sich verhältnismäßig viele Vertiefungen anbringen, ohne das Blatt zu schwächen. Tatsächlich verbleibt zwischen den Vertiefungen auf den Seiten 3 und 4 jeweils ein dünnes Resthäutchen, das mit 9 bezeichnet ist und den Boden der Vertiefungen bildet.

Das Tuch wird herstellerseitig mit dem Substrat getränkt. Als Substrat kommt folgende Mischung in Betracht:
3 % ätherische Öle
4 % Castor Öl
15 % Propylenglykol
0,2 % Kaliumsorbat
77,8 % Wasser dem.

Die ätherischen Öle können wie folgt zusammengesetzt sein:
70 % Eucalyptusöl
10 % Minzöl
8 % Teebaumöl
6 % Rosmarinöl
1 % Bergamottöl
2 % Salbeiöl
3 % Fichtennadelöl

Das vorstehend beschriebene Erzeugnis hat sich als außerordentlich wirksam erwiesen, da das Blatt beispielsweise auf das Gesicht aufgelegt werden kann und über längere Zeiträume unmittelbar in die eingeatmete Luft die Dämpfe des ätherischen Öls abgibt, wodurch eine lange Behandlungsdauer bei einmaliger Anwendung gesichert ist.

## Patentansprüche

1. Humantherapeutisches Erzeugnis zur Behandlung und/oder Vorbeugung von Krankheiten durch Einatmen von Dämpfen ätherischer Öle eines Substrates, gekennzeichnet durch ein Blatt (1) aus Zellstoff mit einer Mehrzahl von regelmäßigen Vertiefungen (5, 8), in denen das Substrat enthalten ist, und durch eine dampfdichte Hülle, in der das mit dem Substrat getränkte Zellstoffblatt (1) enthalten und aus der es zur Therapie entnehmbar ist.

2. Humantherapeutisches Erzeugnis nach Anspruch 1, dadurch gekennzeichnet, daß jede der Vertiefungen (5,8) beiderseits des Blattes (1) angebracht und geschlossen ist.

3. Humantherapeutisches Erzeugnis nach einem oder mehreren der vorausgehenden Ansprüche, dadurch gekennzeichnet, daß die Vertiefungen (5, 8) untereinander im wesentlichen identisch und in gleichen Abständen angeordnet sind.

4. Humantherapeutisches Erzeugnis nach einem oder mehreren der vorausgehenden Ansprüche, dadurch gekennzeichnet, daß die Vertiefungen in der Blattoberfläche (3, 4) geprägt sind.

5. Humantherapeutisches Erzeugnis nach einem oder mehreren der vorausgehenden Ansprüche, dadurch gekennzeichnet, daß die Vertiefungen rechteckig begrenzt und mit ihren Seiten (b, c) parallel zu den Blattkanten (11, 12) in Reihen angeordnet sind.

6. Humantherapeutisches Erzeugnis nach einem oder mehreren der vorausgehenden Ansprüche, dadurch gekennzeichnet, daß die Vertiefungen (5, 8) aufeinanderfolgender Reihen (5, 6) auf Lücke angeordnet sind.

7. Humantherapeutisches Erzeugnis nach einem oder mehreren der vorausgehenden Ansprüche, dadurch gekennzeichnet, daß die Länge (b) der längeren Rechteckseiten der Vertiefungen (5, 8) gleich oder kleiner als 1 mm ist.

8. Humantherapeutisches Erzeugnis nach einem oder mehreren der vorausgehenden Ansprüche, dadurch gekennzeichnet, daß das Zellstoffblatt (1) aus Fluffzellstoff mit einer copolymeren Kunststoffdispersion als Bindemittel besteht.

9. Humantherapeutisches Erzeugnis nach einem oder mehreren der vorausgehenden Ansprüche, dadurch gekennzeichnet, daß der Faserstoff des Blattes (1) ca. 80 Gew.% und das Bindemittel ca. 20 Gew.% ausmachen.

10. Humantherapeutisches Erzeugnis nach einem oder mehreren der vorausgehenden Ansprüche, dadurch gekennzeichnet, daß das Blatt (1) ein Flächengewicht von ca. 80 g/m², eine Naßfestigkeit von ca. 0,28 kN/m in Längs- und ca. 0,25 kN/m in Querrichtung sowie eine Absorption von ca. 480 g/m² aufweist.

11. Humantherapeutisches Erzeugnis nach einem oder mehreren der vorausgehenden Ansprüche, dadurch gekennzeichnet, daß die Dehnung des Blattes (1) im trockenen Zustand ca. 0,28 % in Längs- und ca. 15 % in Querrichtung und die Festigkeit im trockenen Zustand 0,40 kN/m in Längs- und ca. 0,36 kN/m in Querrichtung beträgt.

12. Humantherapeutisches Erzeugnis nach einem oder mehreren der vorausgehenden Ansprüche, dadurch gekennzeichnet, daß das Blatt (1) viereckig mit im wesentlichen gleichen Kantenlängen (a) von ca. 20 cm ausgebildet ist.

13. Humantherapeutisches Erzeugnis nach einem oder mehreren der vorausgehenden Ansprüche, dadurch gekennzeichnet, daß das Blatt (1) in einer Richtung viermal und in der anderen Richtung einmal auf sich selbst gefaltet und gefaltet in der Hülle untergebracht ist.

14. Humantherapeutisches Erzeugnis nach einem oder mehreren der vorausgehenden Ansprüche, dadurch gekennzeichnet, daß das Substrat Öl des Teebaumes und/oder des Eucalyptus und/oder der Minze und/oder Bergamott und/oder der Zitrone und/oder des Rosmarin und/oder der Fichtennadeln und/oder des Salbei aufweist.

15. Humantherapeutisches Erzeugnis nach einem oder mehreren der vorausgehenden Ansprüche, dadurch gekennzeichnet, daß das Substrat folgende Zusammensetzung aufweist: ca. 3 % ätherische Öle, ca. 4 % Castor Öl, ca. 15 % Propylenglykol, ca. 0,2 % Kaliumsorbat, ca. 77,8 % Wasser dem.

16. Humantherapeutisches Erzeugnis nach einem oder mehreren der vorausgehenden Ansprüche, dadurch gekennzeichnet, daß das Substrat folgende Mischung ätherischer Öle enthält: 70 % Eucalyptusöl, 10 % Minzöl, 8 % Teebaumöl, 6 % Rosmarinöl, 1 % Bergamottöl, 2 % Salbeiöl, 3 % Fichtennadelöl.
